# EUROPÄISCHE PATENTSCHRIFT

(11) **EP 1 859 533 B1**
(45) Veröffentlichungstag und Bekanntmachung des Hinweises auf die Patenterteilung: **15.07.2009**
(21) Anmeldenummer: 06707400.5
(22) Anmeldetag: 03.03.2006
(51) Int. Cl.: H04B 1/38, A61B 5/00

(54) **MOBILTELEFON MIT EINGEBAUTEM MESSGERÄT**
MOBILE TELEPHONE PROVIDED WITH A MEASURING DEVICE
TELEPHONE PORTABLE EQUIPE D'UN APPAREIL DE MESURE INTEGRE

(30) Priorität: 03.03.2005 DE 102005009745
(43) Veröffentlichungstag der Anmeldung: 28.11.2007
(73) Patentinhaber: Diabetes.Online AG, 84489 Burghausen (DE)
(72) Erfinder: MERGEN, Wolfgang, 89129 Langenau (DE); ADLASSNIG, Alexander, A-1030 Vienna (AT); SCHNÖLL, Werner, A-3003 Gablitz (AT)
(74) Vertreter: Lang, Johannes
(86) Internationale Anmeldenummer: PCT/EP2006/001968
(87) Internationale Veröffentlichungsnummer: WO 2006/092323

(56) Entgegenhaltungen:
- EP-A- 1 357 780
- WO-A-01/91193
- WO-A-20/04106885

## Beschreibung

### Gebiet der Erfindung

Die vorliegende Erfindung betrifft allgemein ein Mobiltelefon mit integriertem Messgerät bzw. ein in das Mobilfunkgerät integrierbares Messmodul. Die Erfindung betrifft auch einen Teststreifen zur Verwendung in dem Messmodul.

Ein Anwendungsgebiet der Erfindung ist beispielsweise die Blutzuckemessung, bei der der Zuckergehalt in einem Blutstropfen bestimmt wird. Darüber hinaus kann sie jedoch auch auf Messungen jeglicher Art angewendet werden, bei denen regelmäßig bestimmte Analysen vorgenommen werden müssen, etwa auf Schwangerschaftstests von Kühen, bei denen ein Milchtropfen analysiert wird. Hierbei werden in der Regel Teststreifen zur Aufnahme des zu analysierenden Flüssigkeitstropfens benutzt. Ein weiteres Beispiel für ein Anwendungsgebiet ist die Laktatmessung zur objektiven Beurteilung der Ausdauerfähigkeit von Freizeit- und Hochleistungsspontlern. Eine regelmäßige und präzise Laktatmessung erlaubt es dem Sportler - anders als die bloße Ermittlung der Herzfrequenz - immer im optimalen Fettstoffwechselbereich zu trainieren.

Im Folgenden soll das Prinzip der Erfindung insbesondere am Beispiel der Blutzuckermessung erläutert werden.

Zuckerkrankheit (Diabetes mellitus) ist als mit Abstand häufigste Stoffwechselerkrankung sowohl ein medizinisches als auch ein volkswirtschaftliches Problem. Diabetes mellitus ist durch einen krankhaft erhöhten Blutzuckerspiegel und durch die Unfähigkeit des menschlichen Körpers gekennzeichnet, diesen Blutzuckerspiegel in angemessenen Grenzen selbst zu regulieren. Um diese gestörte Regulation zu beheben, bedarf es einer bestimmten Medikation und der regelmäßigen Blutzuckerbestimmung, damit die Medikation auf den aktuellen Blutzuckerspiegel abgestellt werden kann. Zu seltenes und/oder unregelmäßiges Messen führen regelmäßig zu einem zu hohen Blutzuckerspiegel, der wiederum Folgekrankheiten hervorruft, die zu hohen Kosten durch Medikamente, Kranhausaufenthalten und Invalidisierungen führen. Falsche Medikation und schlechte Einstellung des Blutzuckers führen zu schweren Komplikationen und reduzieren die Lebensqualität der Diabetiker enorm.

Mit einer Erhöhung der Messfrequenz können die Medikamente daher präziser dosiert und dadurch die Nebenwirkungen der Zuckerkrankheit deutlich verringert werden. Alle Diabetiker, die Insulin spritzen, berechnen die Dosis nach dem vorher gemessenen Blutzuckerwert. Die Zuverlässigkeit des Messgeräts ist somit ein sehr kritischer Faktor bei der Behandlung.

Aber nicht nur im medizinischen Bereich, sondern auch in vielen anderen Bereichen, wie z.B. in der Landwirtschaft, ist oft eine schnelle und dennoch zuverlässige Messung und Analyse von Daten oft erforderlich.

### Stand der Technik

Der Hauptgrund dafür, dass Diabetiker ihren Blutzucker seltener messen als empfohlen, ist der Umstand, dass sie ihr Messgerät nicht überall bei sich haben. Durch die Integration eines so genannten Blutzucker-Messmoduls in ein Mobiltelefon kann sichergestellt werden, dass der Anwender eine Blutzuckermesseinrichtung ständig mit sich führt, ohne dass dafür ein erhöhter Aufwand in Kauf genommen werden muss.

Aus dem Stand der Technik sind eine Reihe von Vorrichtungen bekannt, bei denen ein Blutzucker-Messmodul mit einem Mobiltelefon verbunden wird oder sich den Platz mit dem Akku des Mobiltelefons teilt, um dadurch eine bessere medizinische Versorgung und Überwachung des Patienten zu gewährleisten. Auch ein direkter Einbau in das Gehäuse außerhalb des Platzes für den Akku wird beschrieben: Die europäische Patentanmeldung EP-A-0 826 963 beschreibt ein Messgerät, das so konstruiert ist, dass es an ein Mobiltelefon angebracht werden kann. Die europäische Patentanmeldung EP-A 0 959 755 beschreibt ein Messgerät, das anstelle des Akku-Packs eines Mobiltelefons angebracht werden kann. Die internationale Patentanmeldung WO-A-01/65810 beschreibt ein Mobiltelefon, in das ein nicht-invasiver Sensor zur Erfassung der Blutzuckerkonzentration eingebaut ist. Da die Blutzuckermessung an einem Touch-Screen erfolgt, sind keine Teststreifen notwendig. Die internationale Patentanmeldung WO-A-2004/106885 beschreibt ein Akku-Pack für Mobiltelefone, das für die Messung des Blutzuckers mit Teststreifen ausgerüstet ist und die Messdaten auf das Display des Mobiltelefons übertragen kann. Auch hier erfolgt die Messung amperometrisch mit Teststreifen, die mindestens eine Arbeits- und eine Referenzelektrode besitzen. Die deutsche Patentanmeldung DE-A-101 02 564 beschreibt ein Mobiltelefon, das in der Lage ist, über einen Teststreifen den Blutzucker seines Besitzers zu messen. Die US-amerikanische Patentanmeldung US-A-2005/0019848 beschäftigt sich mit einem Mobiltelefon, in das ein Blutzucker-Messmodul eingebaut ist. Über einen integrierten Temperatursensor wird versucht, eine Temperaturkorrektur durchzuführen.

Nun ist es aber so, dass, um die Kosten zu senken, in modernen Messgeräten Teststreifen mit sehr kleinen Elektroden verwendet werden. Die bei der Messung des Blutzuckers generierten Ströme liegen deshalb nur mehr im zweistelligen Nanoamper-Bereich. Dazu kommt, dass gleichzeitig auch versucht wird, die Messzeit zu verringern, um die Benutzerfreundlichkeit zu steigern. Moderne Messgeräte liefern im Schnitt schon nach 5 Sekunden den Blutzuckerwert. Beide Faktoren
- geringe Ströme und kurze Messzeiten - machen die Messsysteme jedoch sehr anfällig für Interferenzen durch die Funkmodule im Mobiltelefon. Insbesondere eingehende Anrufe oder Textmeldungen und automatische Anmeldungen und Abmeldungen des Mobiltelefons bei Sendestationen führen zu verstärkten elektromagnetischen Interferenzen, die die Messergebnisse bei Messgeräten, die sich in unmittelbarer Nähe des Mobiltelefons befinden, verfälschen können.

Es gibt eine ganze Reihe von Untersuchungen, die den Einfluss elektromagnetischer Interferenzen von Mobiltelefonen auf medizinische Geräte untersuchen und belegen. Obwohl sich das Ausmaß der beobachteten Störungen im Laufe der Zeit durch das dichtere GSM-Netz, bessere Technologie und nicht zuletzt durch gesetzliche Vorgaben (SAR Grenzwerte) verringert haben, ist laut Experten ein Sicherheitsabstand zwischen Mobiltelefon und medizinischem Gerät von mindestens 10 bis 30 cm einzuhalten, um Fehlfunktionen und falsche Anzeigen zu vermeiden.

Allen Messvorrichtungen, die in den oben aufgelisteten Dokumenten beschriebenen werden ist gemein, dass sie aufgrund ihrer unmittelbaren Nähe zu Mobilfunkgeräten und der dadurch hervorgerufenen elektromagnetischen Interferenzen in den Messmodulen und gegebenenfalls in den Teststreifen Gefahr laufen, verfälschte Messergebnisse hervorzubringen.

### ZUSAMMENFASSUNG DER ERFINDUNG

Es wurde im Stand der Technik bisher jedoch nicht erkannt, dass die Schaltungen eines mit einem Mobilfunkgerät verbundenen Messmoduls und die verwendeten Teststreifen vor den Einwirkungen der von dem Mobiltelefon ausgehenden elektromagnetischen Wellen geschützt werden müssen. Dementsprechend wird auch in keinem der oben genannten Dokumente darauf eingegangen, wie ein solcher Schutz erreicht werden könnte.

Aufgabe der Erfindung ist es demnach, ein Messgerät zur Verfügung zu stellen, das es dem Benutzer einerseits erlaubt, auf komfortable Weise regelmäßige Messungen durchzuführen, dabei jedoch andererseits eine hohe Zuverlässigkeit bezüglich der Korrektheit der Messergebnisse trotz der unmittelbaren Nähe des Messgeräts zu den Funkmodulen eines Mobilfunkgeräts aufweist. Die Aufgabe wird durch die Vorrichtungen gelöst, die in den unabhängigen Ansprüchen 1, 11, 12 und 14 definiert sind. Weitere, bevorzugte Ausführungsformen der Erfindung sind in den abhängigen Ansprüchen definiert.

Eine bevorzugte Ausführungsform der Erfindung ist ein Mobilfunkgerät, in das ein Messmodul eingebaut ist, wobei das Messmodul durch eine oder mehrere Metallschichten, die das Messmodul umgeben, gegen elektromagnetische Wellen abgeschirmt wird, die durch das Mobilfunkgerät erzeugt werden.

Dies hat den Vorteil, dass die Abschirmung gegen elektromagnetische Wellen auf einfache und kostengünstige Weise bewirkt wird.

Gemäß einer weiteren bevorzugten Ausführungsform der Erfindung sind die Metallschichten mit einem festen Bezugspotential verbunden, welches vorzugsweise die Masse des Mobilfunkgerätes ist.

Dies hat den Vorteil, dass der Wirkungsgrad der Abschirmung beträchtlich erhöht wird.

Gemäß einer weiteren bevorzugten Ausführungsform der Erfindung ist das verwendete Metall Aluminium, Kupfer, Stahl, Zink, Nickel, Blei, Gold oder eine Legierung dieser Metalle.

Dies hat den Vorteil, dass die Abschirmung auf effiziente und/oder kostengünstige Weise bewirkt wird

Gemäß einer weiteren bevorzugten Ausführungsform der Erfindung teilt sich das Messmodul im Mobilfunkgerät einen Raum mit der Batterie des Mobilfunkgeräts.

Dies hat den Vorteil, dass für den Einbau eines Messgeräts in ein beliebiges Mobilfunkgerät dessen äußeres Design unverändert bleiben kann und lediglich das Design des Akkupacks verändert werden muss.

Gemäß einer weiteren bevorzugten Ausführungsform der Erfindung ist das Messmodul in den Akku-Pack des Mobilfunkgeräts eingebaut.

Dies hat den Vorteil, dass die Messdaten über die vorhandene serielle Datenschnittstelle zwischen dem Mobilfunkgerät und seinem Akkupack ausgetauscht werden können.

Gemäß der Erfindung weist das Mobilfunkgerät eine Öffnung auf, durch die ein Teststreifen in das Messmodul eingeführt werden kann, vorzugsweise an einer Längsseite des Mobilfunkgeräts, und zwar vorzugsweise an der linken Längsseite.

Dies hat den Vorteil, dass das Messgerät mit seiner länglichen Geometrie leicht oberhalb oder unterhalb der Batterie eingebaut werden kann und dass Rechtshändern das Aufbringen des Blutstropfens erleichtert wird.

Eine weitere bevorzugte Ausführungsform der Erfindung ist ein Teststreifen zur Verwendung in einem Messmodul, das in ein Mobilfunkgerät eingebaut ist, wobei der Teststreifen durch eine oder mehrere Metallschichten, die auf den Teststreifen aufgebracht werden, gegen elektromagnetische Wellen abgeschirmt ist, die durch das Mobilfunkgerät erzeugt werden.

Dies hat den Vorteil, dass die Abschirmung gegen elektromagnetische Wellen auf einfache und kostengünstige Weise bewirkt wird.

Gemäß einer weiteren bevorzugten Ausführungsform der Erfindung wird eine der Metallschichten unterhalb des Messstreifens aufgebracht.

Dies hat den Vorteil, dass die Abschirmungsschicht leicht aufgebracht werden kann und eine große Abschirmungsfläche bietet.

Gemäß einer weiteren bevorzugten Ausführungsform der Erfindung wird eine der Metallschichten, die vorzugsweise aus dem selben Metall wie die Messelektroden besteht, und zwar vorzugsweise aus Kupfer oder einer Kupferlegierung, auf die freien Flächen in der Schicht der Messelektroden und Leiterbahnen des Messstreifens aufgebracht.

Dies hat den Vorteil, dass der Teststreifen kostengünstig mit der Leiterplatten-Technologie hergestellt werden kann.

Gemäß einer weiteren bevorzugten Ausführungsform der Erfindung werden die Metallschichten mit einem festen. Bezugspotential verbunden, das vorzugsweise die Masse des Mobilfunkgeräts ist.

Dies hat den Vorteil, dass der Wirkungsgrad der Abschinnung beträchtlich erhöht wird.

Gemäß einer weiteren bevorzugten Ausführungsform der Erfindung dient das Messmodul zur Messung des Blutzuckers.

Dies hat den Vorteil, dass ein Diabetiker, der ohnehin sein Mobilfunkgerät immer bei sich trägt, mit diesem stets verlässliche Blutzuckertests machen kann.

### KURZE BESCHREIBUNG DER ZEICHNUNG

Die obige und andere Aufgaben, Aspekte und Vorteile der Erfindung werden mit der folgenden detaillierten Beschreibung der bevorzugten Ausführungsformen der

Erfindung besser verstanden werden, in der Bezug auf die Zeichnung genommen wird, in der:
- Fig. 1: ein Mobiltelefon gemäß einer Ausführungsform der Erfindung in Vorderansicht zeigt, in das ein Blutzucker-Messmodul eingebaut ist;
- Fig. 2: das Mobiltelefon aus Fig. 1 in Seitenansicht zeigt;
- Fig. 3: ein Akku-Pack mit eingebautem Blutzucker-Messmodul als eine weitere Ausführungsform der Erfindung zeigt;
- Fig. 4: in Explosionsansicht den Aufbau des Akku-Packs aus Fig. 3 zeigt;
- Fig. 5: in Explosionsansicht den Aufbau eines Teststreifens als eine weitere Ausführungsform der Erfindung zeigt;
- Fig. 6: einen Querschnitt durch den Teststreifen aus Fig. 5 zeigt.

### DETAILLIERTE BESCHREIBUNG DER BEVORZUGTEN AUSFÜHRUNGSFORMEN DER ERFINDUNG

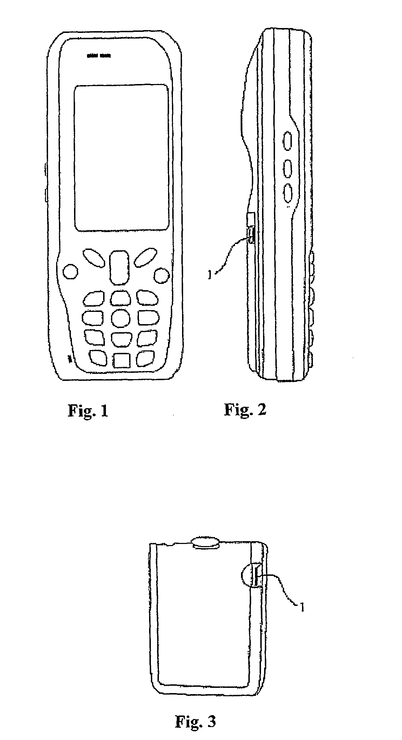

Fig. 1 zeigt ein Mobiltelefon, in das ein Messgerät, beispielsweise ein Blutzucker-Messgerät eingebaut ist, in Vorderansicht. Beim Entwurf für Blutzuckermessgeräte sollte speziell auf ein großes Display und einfache Bedienung durch intuitive Menüführung und möglichst großen Abstand zwischen den Tasten geachtet werden, weil die Prävalenz von Diabetes mellitus mit dem Alter sehr stark ansteigt. Design und Bedienung des Mobiltelefons sollte deshalb an die Bedürfnisse und Anforderungen der Zielgruppe angepasst sein. Ein großes Display ist besonders auf Grund der häufigen mikrovaskulären Komplikationen im Bereich der Augen vorteilhaft (fast alle Typ-I und mehr als 60% der Typ-II Diabetiker entwickeln im Laufe der Erkrankung auch eine Erkrankung der Netzhaut, genannt Retinopathie). Eine derartige Ausgestaltung kann auch in anderen Bereichen von Vorteil sein, wo unter schwierigen Arbeitsbedingungen Messergebnisse abgelesen werden müssen. Derartige Ausgestaltungen sind jedoch kein Erfordernis der Erfindung. Es ist im Gegenteil ein wesentlicher Vorteil der Erfindung, das sie mit jedem konventionellen serienmäßigen Mobiltelefon benutzt werden kann.

Das Messmodul kann an einer beliebigen Stelle innerhalb des Gehäuses des Mobiltelefons angeordnet werden. Am einfachsten und kostengünstigsten ist jedoch eine Integration in den Akku-Pack des Mobiltelefons, weil die meisten Mobiltelefone bereits über eine serielle Datenschnittstelle mit dem Akku-Pack kommunizieren. Die Integration erfordert deshalb auf der Seite des Mobiltelefons lediglich eine Anpassung der Software, um die vom Messmodul gesendeten Daten darzustellen und zu verarbeiten. Standardmäßige Mobiltelefone, wie sie üblicherweise auf dem Markt sind, lassen sich so durch ein einfaches Software-Update und dem entsprechenden Akku-Pack zu Messgeräten umfunktionieren.

Fig. 2 zeigt das Mobiltelefon aus Fig. 1 in Seitenansicht. Auf der linken Längsseite ist im Akku-Pack des Mobiltelefons eine Öffnung erkennbar, die den Teststreifenport 1 des Messmoduls darstellt. Das Messmodul ist von außen nur durch diesen Teststreifenport 1 sichtbar.

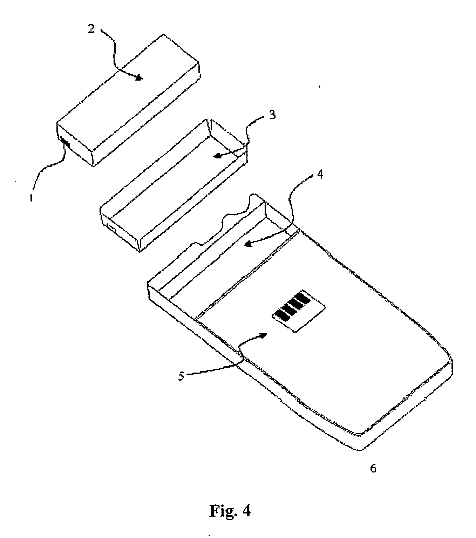

Fig. 3 zeigt den Akku-Pack des Mobiltelefons aus den Fig. 1 und 2 separat in Ansicht auf die Rückseite des Mobiltelefons. In der Explosionsansicht dieses Akku-Packs in Fig. 4 ist eine bevorzugte Anordnung des Messmoduls 2 im Akku-Pack 6 erkennbar. Wegen der länglichen Form des Messmoduls 2 und der Geometrie der im Akku-Pack 6 enthaltenen Batterien 5 wird das Messmodul 2 oberhalb der Batterien 5 angeordnet. Der Akku-Pack 6 ist so dimensioniert, dass oberhalb der Batterien 5 ein ausreichend großer Freiraum 4 für das Messmodul 2 bleibt.

Bei einer solchen Anordnung des Messmoduls 2 kann der Teststreifenport 1 nicht wie bei herkömmlichen separat erhältlichen Messgeräten an der oberen oder unteren Seitenfläche platziert werden. In einer bevorzugten Ausführungsform liegt der Teststreifenport 1 daher auf an der linken Seitenfläche des Mobilfunkgeräts (bei Ansicht von vorne). Da die meisten Menschen Rechtshänder sind und sich daher zur Gewinnung des für die Messung erforderlichen Blutstropfens in einen Finger der linken Hand stechen, ermöglicht diese Anordnung ein vereinfachtes Aufbringen des Blutstropfens auf den Teststreifen. Umständliches hantieren über dem Mobiltelefon wird dadurch ebenso vermieden wie das Benutzen der Tastatur mit dem nachblutenden Finger im Anschluss an die Messung.

Das Messmodul 2 ist von einer Metallschicht 3 umgeben, die zur Abschirmung der von dem Mobilfunkgerät ausgehenden elektromagnetischen Wellen dient, welche störende Interferenzen im Messmodul und dem in das Messmodul eingeführen Teststreifen hervorrufen können. Unter einer Metallschicht wird hier eine Schicht verstanden, in der unter anderem Metall enthalten ist; gegebenenfalls kann es sich dabei auch um ein Metallgitter handeln. Obwohl eine vollständige Abschirmung aller elektromagnetischen Wellen nie erzielt werden kann, kann jedoch mit einer Abschirmung zumindest eine fehlerfreie Messung trotz der ansonsten fatalen Nähe zur Antenne und dem Funkmodul gewährleistet werden. Der Wirkungsgrad der Abschirmung sollte vorzugsweise mindestens 97% betragen.

Die Metallschicht 3 umgibt das Messmodul 2 vorzugsweise von allen Seiten, um eine möglichst effiziente Abschirmungswirkung zu erzielen. Die Abschirmung kann auch mit mehreren, nicht notwendiger Weise zusammenhängenden Metallschichten realisiert werden. Zum Beispiel kann wie in Fig. 4 eine Metallschicht 3 vor dem Einbau des Messmoduls 2 angebracht werden, die auf der einen Seite (in Fig. 4 der oberen Seite) offen ist, um den Einbau des Messmoduls zu erlauben. Die Abschirmung auf der offenen Seite kann etwa dadurch realisiert werden, dass auf das Messmodul 2 auf dieser Seite eine (von der Metallschicht 3 separate) Metallschicht aufgebracht ist, zum Beispiel durch Aufdampfen.

Die Metallschichten sind vorzugsweise aus Aluminium, Kupfer, Stahl, Zink, Blei, Silber, Gold oder Legierungen dieser Metalle. Zur Erhöhung des Abschirmungsgrades werden die Metallschichten vorzugsweise mit einem festen Bezugspotential, etwa der Masse des Mobilfunkgeräts, verbunden. Eine solche Verbindung wird zum Beispiel durch Anbringen eines leitenden Silikongummis bewerkstelligt, der beim Befestigen des Akku-Packs am Mobiltelefon das Abschirmgehäuse mit einer Masseleitung der Hauptplatine verbindet.

Um die Korrektheit der Messungen zu gewährleisten, reicht es unter Umständen jedoch nicht aus, das im Mobilfunkgerät eingebaute Messmodul abzuschirmen, sondern es muss zusätzlich der für die Messung verwendete Teststreifen abgeschirmt werden, wie er etwa für die amperometrische Bestimmung des Blutzuckergehalts (der elektrische Widerstand des Bluts ändert sich mit dem Blutzuckergehalt) verwendet wird.

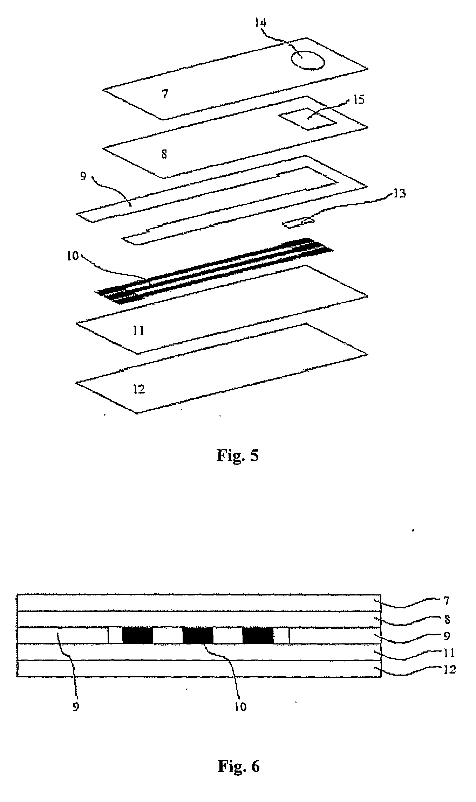

Fig. 5 zeigt den bevorzugten Aufbau eines abgeschirmten Teststreifens. Die Sensorik 10 und eine obere Abschirmung 9 werden in ein- und demselben Arbeitsschritt auf das Substrat 11 aufgebracht. Auf der Unterseite des Sensorstreifens befindet sich eine dünne untere Abschirmungsschicht, vorzugsweise aus Kupfer. Nach dem Aufdrucken der Enzymschicht 13 auf die Arbeitselektrode werden ein Abstandshalter 8 und eine Deckschicht 7 aufgeklebt. Im Abstandshalter ist ein Reaktionsraum 15 ausgespart, in dem das durch ein spezielles Auftragsfenster 14 zugeführte Blut mit der in der Enzymschicht befindlichen Glukoseoxidase reagiert und ein Messsignal liefert.

Fig. 6 ist eine Ansicht im Querschnitt des Teststreifens aus Fig. 5, in der sich folgende Schichten erkennen lassen:
- Deckschicht 7
- Abstandsschicht 8
- obere Abschirmungsschicht 9 und Leitungsbahnen 10
- Substratschicht 11
- untere Abschirmungsschicht 12

Herkömmliche amperometrische Teststreifen verwenden Graphitelektroden, bei denen zum Beispiel das Herstellen einer Abschirmung auf der Streifenunterseite nur mit sehr hohem technischen Aufwand möglich wäre. Zudem müsste die Schichtdicke der im Siebdruckverfahren aufgebrachten Graphitabschirmung etwa 40 Mal dicker sein als eine Kupferschicht mit derselben Effizienz. Die Herstellung abgeschirmter Teststreifen für amperometrische Bestimmung des Blutzuckergehalts mit Graphitelektroden wäre daher mit sehr großem Aufwand verbunden und auch teuer.

Der Teststreifen wird daher bevorzugt mit Hilfe der Leiterplatten-Technologie hergestellt. Die Elektroden und Leiterbahnen 10 sind aus Kupfer. Die Leiterbahnen- und Elektrodenregion wird durch einfache Verkupferung der freien Fläche auf dem Substrat 11 abgeschirmt. Darüber hinaus sorgt eine dünne Kupferschicht 12 auf der Unterseite des Teststreifens (unterhalb der Elektroden) für zusätzliche Abschirmung vor elektromagnetischen Wellen. Die einheitliche Verwendung von Kupfer für Elektroden, Leiterbahnen 10 und Abschirmungsschichten 9, 12 hat den Vorteil, dass sowohl die für die Messung notwendigen Elemente des Sensorstreifens als auch die Abschirmung in einem Arbeitsschritt und kostengünstig hergestellt werden können. Die Verwendung anderer Metalle zur Realisierung der Abschirmungsschichten, insbesondere Stahl, Zink, Blei, Silber, Gold oder Legierungen dieser Metalle, einschließlich Kupferlegierungen, ist jedoch denkbar.

Die Teststreifen gemäß der bevorzugten Ausführungsform sind breiter als konventionelle Teststreifen. Dadurch ist genügend Platz, um sowohl die Leiterbahnen als auch den Teststreifen abzuschirmen. Infolgedessen muss auch der Teststreifenport des Messmoduls etwas breiter dimensioniert werden.

Die Metallschichten zur Abschirmung des Teststreifens sind, wenn der Teststreifen in das Messmodul eingeführt ist, mit einem festen Bezugspotential verbunden, etwa der Masse des Mobilfunkgeräts, um einen höheren Abschirmungsgrad zu erzielen.

Es ist ein weiterer Vorteil der Erfindung, dass das Mobiltelefon die gemessenen Werte direkt auf seinem Display anzeigen kann. Darüber hinaus kann das Mobiltelefon aber auch direkt als Kommunikationsgerät dienen, über das die Messwerte an eine Empfangsstation zur Auswertung übermittelt werden können. Im Falle der Nutzung für Blutzuckertests kann beispielsweise bei lebensbedrohlicher Über- oder Unterzuckerung automatisch Hilfe herbeigerufen werden. Dabei kann über die Funkzelle, in der sich das Mobiltelefon befindet, der Patient lokalisiert werden. Die Messdaten können auch direkt zur Archivierung und Auswertung an eine entsprechende Datenbank bei einem Arzt oder Krankenhaus gesendet werden.

## Patentansprüche

1. Mobiltelefon, in das ein Messmodul (2) eingebaut ist, in welches zur Messung ein Teststreifen durch eine Öffnung (1) eingeführt werden kann, **dadurch gekennzeichnet, dass** das Messmodul (2) gegen elektromagnetische Wellen abgeschirmt ist, die durch das Mobiltelefon erzeugt werden, und dass das Mobiltelefon und/oder das Messmodul (2) dafür eingerichtet sind, dass eine aus einer oder mehreren Metallschichten (9, 12) bestehende Abschirmung des Teststreifens mit einem festen Bezugspotential verbunden wird, wenn der Teststreifen in das Messmodul (2) eingeführt wird.

2. Mobiltelefon gemäß Anspruch 1, **dadurch gekennzeichnet, dass** die Abschirmung des Messmoduls (2) durch eine oder mehrere Metallschichten (3) bewirkt wird, die das Messmodul (2) umgeben.

3. Mobiltelefon gemäß Anspruch 2, **dadurch gekennzeichnet, dass** die Metallschichten (3) der Abschirmung des Messmoduls (2) mit einem festen Bezugspotential verbunden sind.

4. Mobiltelefon gemäß einem der Ansprüche 1 bis 3, **dadurch gekennzeichnet, dass** das feste Bezugspotential die Masse des Mobiltelefons ist.

5. Mobiltelefon gemäß einem der Ansprüche 2 bis 4, **dadurch gekennzeichnet, dass** das verwendete Metall Aluminium, Kupfer, Stahl, Zink, Nickel, Blei, Gold oder eine Legierung dieser Metalle ist.

6. Mobiltelefon gemäß einem der vorhergehenden Ansprüche, **dadurch gekennzeichnet, dass** sich das Messmodul (2) im Mobiltelefon einen Raum mit der Batterie (5) des Mobiltelefons teilt.

7. Mobiltelefon gemäß Anspruch 6, **dadurch gekennzeichnet, dass** das Messmodul (2) in den Akku-Pack (6) des Mobiltelefons eingebaut ist.

8. Mobiltelefon gemäß einem der vorhergehenden Ansprüche, **dadurch gekennzeichnet, dass** die Öffnung (1) an einer der Längsseiten des Mobiltelefons angeordnet ist.

9. Mobiltelefon gemäß Anspruch 8, **dadurch gekennzeichnet, dass** die Längsseite die, von vorne gesehen, linke Längsseite des Mobiltelefons ist.

10. Mobiltelefon gemäß einem der vorhergehenden Ansprüche, **dadurch gekennzeichnet, dass** das Messmodul (2) zur Messung des Blutzuckers dient.

11. Messmodul (2) für den Einbau in ein Mobiltelefon, in das zur Messung ein Teststreifen durch eine Öffnung (1) eingeführt werden kann, **dadurch gekennzeichnet, dass** das Messmodul (2) durch eine oder mehrere Metallschichten (9, 12) umgeben ist, um es gegen elektromagnetische Wellen abzuschirmen, die durch das Mobiltelefon erzeugt werden und dass das Messmodul (2) dafür eingerichtet ist, dass eine aus einer oder mehreren Metallschichten (9, 12) bestehende Abschirmung des Teststreifens mit einem festen Bezugspotential verbunden wird, wenn der Teststreifen in das Messmodul (2) eingeführt wird.

12. Akkupack (6) für ein Mobiltelefon, das ein Messmodul (2) gemäß Anspruch 11 umfasst.

13. Akkupack (6) gemäß Anspruch 12, **dadurch gekennzeichnet, dass** das Messmodul (2) zur Messung des Blutzuckers dient.

14. Teststreifen zur Verwendung in einem Messmodul (2), das in ein Mobiltelefon eingebaut ist, **dadurch gekennzeichnet, dass** der Teststreifen gegen elektromagnetische Wellen abgeschirmt ist, die durch das Mobiltelefon erzeugt werden.

15. Teststreifen gemäß Anspruch 15, **dadurch gekennzeichnet, dass** die Abschirmung durch eine oder mehrere Metallschichten (9, 12) bewirkt wird, die auf den Teststreifen aufgebracht werden.

16. Teststreifen gemäß Anspruch 15, **dadurch gekennzeichnet, dass** eine der Metallschichten (12) unterhalb des Messstreifens aufgebracht wird.

17. Teststreifen gemäß Anspruch 15 oder 16, **dadurch gekennzeichnet, dass** eine der Metallschichten (9) auf die freien Flächen in der Schicht der Messelektroden und Leiterbahnen (10) des Messstreifens aufgebracht wird.

18. Teststreifen gemäß Anspruch 17, **dadurch gekennzeichnet, dass** die Metallschicht (9) aus dem selben Metall wie die Messelektroden (10) besteht.

19. Teststreifen gemäß Anspruch 18, **dadurch gekennzeichnet, dass** das Metall Kupfer oder eine Kupferlegierung ist.

20. Teststreifen gemäß einem der Ansprüche 15 bis 19, **dadurch gekennzeichnet, dass** die Metallschichten (9, 12) mit einem festen Bezugspotential verbunden werden.

21. Teststreifen gemäß Anspruch 20, **dadurch gekennzeichnet, dass** das feste Bezugspotential die Masse des Mobiltelefons ist.

22. Teststreifen gemäß einem der Ansprüche 14 bis 21, **dadurch gekennzeichnet, dass** er zur Verwendung in einem Blutzucker-Messmodul dient.

## Claims

1. Mobile telephone with an integral measuring module (2), into which for the measurement a test strip can be introduced through an opening (1),
**characterized in that** the measuring module (2) is shielded against electromagnetic waves generated by the mobile telephone, and that the mobile telephone and/or the measuring module (2) are adapted such that a shielding consisting of one or more metal layers (9, 12) of the test strip is connected to a fixed reference potential when the test strip is introduced into the measuring module (2).

2. Mobile telephone according to claim 1, **characterized in that** the shielding of the measuring module (2) is effected by one or more metal layers (3) surrounding the measuring module (2).

3. Mobile telephone according to claim 2, **characterized in that** the metal layers (3) of the shielding of the measuring module (2) are connected to a fixed reference potential.

4. Mobile telephone according to any one of claims 1 to 3, **characterized in that** the fixed reference potential is the ground of the mobile telephone.

5. Mobile telephone according to any one of claims 2 to 4, **characterized in that** the employed metal is aluminum, copper, steel, zinc, nickel, lead, gold or an alloy of said metals.

6. Mobile telephone according to any one of the preceding claims, **characterized in that** the measuring module (2) in the mobile telephone shares a space with the battery (5) of the mobile telephone.

7. Mobile telephone according to claim 6, **characterized in that** the measuring module (2) is installed in the battery pack (6) of the mobile telephone.

8. Mobile telephone according to any of the preceeding claims, **characterized in that** the opening (1) is arranged on one of the longitudinal sides of the mobile telephone.

9. Mobile telephone according to claim 8, **characterized in that** the longitudinal side is the left-hand longitudinal side, viewed from the front, of the mobile telephone.

10. Mobile telephone according to any one of the preceding claims, **characterized in that** the measuring module (2) serves to measure the blood sugar.

11. Measuring module (2) for installation in a mobile telephone into which for the measurement a test strip can be introduced through an opening (1),
**characterized in that** the measuring module (2) is surrounded by one or more metal layers (9, 12) in order to shield said measuring module against electromagnetic waves generated by the mobile telephone and the measuring module (2) is adapted such that a shielding consisting of one or more metal layers (9, 12) of the test strip is connected to a fixed reference potential when the test strip is introduced into the measuring module (2).

12. Battery pack (6) for a mobile telephone, said battery pack comprising a measuring module (2) according to claim 11.

13. Battery pack (6) according to claim 12, **characterized in that** the measuring module (2) serves to measure the blood sugar.

14. Test strip for use in a measuring module (2) installed in a mobile telephone, **characterized in that** the test strip is shielded against electromagnetic waves generated by the mobile telephone.

15. Test strip according to claim 14, **characterized in that** the shielding is effected by one or more metal layers (9, 12) applied to the test strip.

16. Test strip according to claim 15, **characterized in that** one of the metal layers (12) is applied below the test strip.

17. Test strip according to claim 15 or 16, **characterized in that** one of the metal layers (9) is applied to the free surfaces in the layer of the measuring electrodes and conductors (10) of the test strip.

18. Test strip according to claim 17, **characterized in that** the metal layer (9) is of the same metal as the measuring electrodes (10).

19. Test strip according to claim 18, **characterized in that** the metal is copper or a copper alloy.

20. Test strip according to any one of claims 15 to 19, **characterized in that** the metal layers (9, 12) are connected to a fixed reference potential.

21. Test strip according to claim 20, **characterized in that** the fixed reference potential is the ground of the mobile telephone.

22. Test strip according to any one of claims 14 to 21, **characterized in that** said test strip serves for use in a blood sugar measuring module.

## Revendications

1. Téléphone mobile dans lequel est intégré un module de mesure (2) dans lequel une bandelette de test peut être insérée par une ouverture (1) aux fins de mesure, **caractérisé en ce que** le module de mesure (2) est blindé contre les ondes électromagnétiques produites par le téléphone mobile, et **en ce que** le téléphone mobile et/ou le module de mesure (2) sont conçus de façon qu'un blindage de la bandelette de test, constitué d'une ou plusieurs couches métalliques (9, 12), soit relié à un potentiel de référence fixe lorsque la bandelette de test est introduite dans le module de mesure (2).

2. Téléphone mobile selon la revendication 1, **caractérisé en ce que** le blindage du module de mesure (2) est réalisé par l'intermédiaire d'une ou plusieurs couches métalliques (3) qui entourent le module de mesure (2).

3. Téléphone mobile selon la revendication 2, **caractérisé en ce que** les couches métalliques (3) du blindage du module de mesure (2) sont reliées à un potentiel de référence fixe.

4. Téléphone mobile selon une des revendications 1 à 3, **caractérisé en ce que** le potentiel de référence fixe est la masse du téléphone mobile.

5. Téléphone mobile selon une des revendications 2 à 4, **caractérisé en ce que** le métal employé est de l'aluminium, du cuivre, de l'acier, du zinc, du nickel, du plomb, de l'or ou un alliage de ces métaux.

6. Téléphone mobile selon une des revendications précédentes, **caractérisé en ce que** le module de mesure (2) partage dans le téléphone mobile un espace avec la batterie (5) du téléphone mobile.

7. Téléphone mobile selon la revendication 6, **caractérisé en ce que** le module de mesure (2) est intégré au bloc d'accumulateurs (6) du téléphone mobile.

8. Téléphone mobile selon une des revendications précédentes, **caractérisé en ce que** l'ouverture (1) est disposée sur un des grands côtés du téléphone mobile.

9. Téléphone mobile selon la revendication 8, **caractérisé en ce que** le grand côté est le grand côté du téléphone mobile situé à gauche quand on regarde celui-ci de face.

10. Téléphone mobile selon une des revendications précédentes, **caractérisé en ce que** le module de mesure (2) sert à mesurer le sucre sanguin.

11. Module de mesure (2) à intégrer dans un téléphone mobile, dans lequel une bandelette de test peut être insérée par une ouverture (1) aux fins de mesure, **caractérisé en ce que** le module de mesure (2) est entouré d'une ou plusieurs couches métalliques (9, 12) pour le blinder contre les ondes électromagnétiques produites par le téléphone mobile, et **en ce que** le module de mesure (2) est conçu de façon qu'un blindage de la bandelette de test, constitué d'une ou plusieurs couches métalliques (9, 12), soit relié à un potentiel de référence fixe lorsque la bandelette de test est introduite dans le module de mesure (2).

12. Bloc d'accumulateurs (6) pour un téléphone mobile qui comprend un module de mesure (2) selon la revendication 11.

13. Bloc d'accumulateurs (6) selon la revendication 12, **caractérisé en ce que** le module de mesure (2) sert à mesurer le sucre sanguin.

14. Bandelette de test à employer dans un module de mesure (2) qui est intégré à un téléphone mobile, **caractérisée en ce que** la bandelette de test est blindée contre les ondes électromagnétiques produites par le téléphone mobile.

15. Bandelette de test selon la revendication 15, **caractérisée en ce que** le blindage est réalisé par l'intermédiaire d'une ou plusieurs couches métalliques (9, 12) qui sont appliquées sur la bandelette de test.

16. Bandelette de test selon la revendication 15, **caractérisée en ce qu'**une des couches métalliques (12) est appliquée sous la bandelette de mesure.

17. Bandelette de test selon la revendication 15 ou 16, **caractérisée en ce qu'**une des couches métalliques (9) est appliquée sur les surfaces libres dans la couche des électrodes de mesure et des pistes conductrices (10) de la bandelette de mesure.

18. Bandelette de test selon la revendication 17, **caractérisée en ce que** la couche métallique (9) est constituée du même métal que les électrodes de mesure (10).

19. Bandelette de test selon la revendication 18, **caractérisée en ce que** le métal est du cuivre ou un alliage de cuivre.

20. Bandelette de test selon une des revendications 15 à 19, **caractérisée en ce que** les couches métalliques (9, 12) sont reliées à un potentiel de référence fixe.

21. Bandelette de test selon la revendication 20, **caractérisée en ce que** le potentiel de référence fixe est la masse du téléphone mobile.

22. Bandelette de test selon une des revendications 14 à 21, **caractérisée en ce qu'**elle est employée dans un module de mesure du sucre sanguin.
